# EUROPEAN PATENT SPECIFICATION

(11) **EP 2 892 575 B1**
(45) Date of publication and mention of the grant of the patent: **08.07.2020**
(21) Application number: 13762683.4
(22) Date of filing: 05.09.2013
(51) Int. Cl.: A61L 27/26, C08B 37/08, C08H 1/00, C08L 5/08, C08L 89/00

(54) **HYALURONIC ACID/COLLAGEN- BASED DERMAL FILLER COMPOSITIONS AND METHODS FOR MAKING SAME**
DERMALE FÜLLSTOFFZUSAMMENSETZUNGEN AUF DER BASIS VON HYALURONSÄURE/COLLAGEN UND VERFAHREN ZU IHRER HERSTELLUNG
COMPOSITIONS DE COMBLEMENT DERMIQUE À BASE D'ACIDE HYALURONIQUE/COLLAGÈNE ET PROCÉDÉS DE FABRICATION ASSOCIÉS

(30) Priority: 06.09.2012 US 201213605565
(43) Date of publication of application: 15.07.2015
(73) Proprietor: ALLERGAN, INC., Irvine, CA 92612 (US)
(72) Inventor: POLLOCK, Jacob, F., Charleston, West Virginia 25314 (US); YU, Xiaojie, Irvine, California 92602 (US); MANESIS, Nicholas, J., Summerland, California 93067 (US)
(74) Representative: Hoffmann Eitle
(86) International application number: PCT/US2013/058140
(87) International publication number: WO 2014/039607

(56) References cited:
- EP-A1- 0 656 215
- EP-A1- 1 532 991
- EP-A2- 0 671 165
- WO-A2-2009/048930
- US-A1- 2005 186 673
- US-A1- 2006 189 516
- PARK S-N ET AL: "Characterization of porous collagen/hyaluronic acid scaffold modified by 1-ethyl-3-(3-dimethylaminopropyl)carbodiim ide cross-linking", BIOMATERIALS, ELSEVIER SCIENCE PUBLISHERS BV., BARKING, GB, vol. 23, no. 4, 15 February 2002 (2002-02-15), pages 1205-1212, XP004348139, ISSN: 0142-9612, DOI: 10.1016/S0142-9612(01)00235-6
- PARK S ET AL: "Biological characterization of EDC-crosslinked collagen-hyaluronic acid matrix in dermal tissue restoration", BIOMATERIALS, ELSEVIER SCIENCE PUBLISHERS BV., BARKING, GB, vol. 24, no. 9, 1 April 2003 (2003-04-01), pages 1631-1641, XP004404219, ISSN: 0142-9612, DOI: 10.1016/S0142-9612(02)00550-1

## Description

### BACKGROUND

The present invention generally relates to dermal filler compositions, and more specifically relates to injectable dermal filler compositions including crosslinked hyaluronic acid and collagen. Hyaluronic acid and collagen are key structural components of human tissues. These biopolymers have been widely used to construct tissue engineering scaffolds and materials for cell culturing and regenerative medicine.

Skin aging is a progressive phenomenon, occurs over time and can be affected by lifestyle factors, such as alcohol consumption, tobacco and sun exposure. Aging of the facial skin can be characterized by atrophy, slackening, and fattening. Atrophy corresponds to a massive reduction of the thickness of skin tissue. Slackening of the subcutaneous tissues leads to an excess of skin and ptosis and leads to the appearance of drooping cheeks and eye lids. Fattening refers to an increase in excess weight by swelling of the bottom of the face and neck. These changes are typically associated with dryness, loss of elasticity, and rough texture.

Hyaluronan, also known as hyaluronic acid (HA) is a non-sulfated glycosaminoglycan that is distributed widely throughout the human body in connective, epithelial, and neural tissues. Hyaluronan is abundant in the different layers of the skin, where it has multiple functions such as, e.g., to ensure good hydration, to assist in the organization of the extracellular matrix, to act as a filler material; and to participate in tissue repair mechanisms. However, with age, the quantity of hyaluronan, collagen, elastin, and other matrix polymers present in the skin decreases. For example, repeated exposed to ultra violet light, e.g., from the sun, causes dermal cells to both decrease their production of hyaluronan as well as increase the rate of its degradation. This hyaluronan loss results in various skin conditions such as, e.g., imperfects, defects, diseases and/or disorders, and the like. For instance, there is a strong correlation between the water content in the skin and levels of hyaluronan in the dermal tissue. As skin ages, the amount and quality of hyaluronan in the skin is reduced. These changes lead to drying and wrinkling of the skin.

Dermal fillers are useful in treating soft tissue condition and in other skin therapies because the fillers can replace lost endogenous matrix polymers, or enhance/facilitate the function of existing matrix polymers, in order to treat these skin conditions. In the past, such compositions have been used in cosmetic applications to fill wrinkles, lines, folds, scars, and to enhance dermal tissue, such as, e.g., to plump thin lips, or fill-in sunken eyes or shallow cheeks. Earlier dermal filler products generally were made of collagens. One common matrix polymer used in modern dermal filler compositions is hyaluronan. Because hyaluronan is natural to the human body, it is a generally well tolerated and a fairly low risk treatment for a wide variety of skin conditions.

Originally, compositions comprising hyaluronan where made from naturally-occurring polymers, which exist in an uncrosslinked state. Although exhibiting excellent biocompatibility and affinity for water molecules, naturally-occurring hyaluronan exhibits poor biomechanical properties as a dermal filler. One primary reason is that because this polymer is uncrosslinked, it is highly soluble and, as such, is cleared rapidly when administered into a skin region. This *in vivo* clearance is primarily achieved by rapid degradation of the polymers, principally enzymatic degradation via hyaluronidase and chemical degradation via free-radicals. Thus, while still in commercial use, compositions comprising uncrosslinked hyaluronan polymers tend to degrade within a few days after administration and thus require fairly frequent reinjection to maintain their skin improving effect.

To minimize the effect of these *in vivo* degradation pathways, matrix polymers are crosslinked to one another to form a stabilized hydrogel. Because hydrogels comprising crosslinked matrix polymers are a more solid substance, dermal fillers comprising such hydrogels remain in place at the implant site longer. In addition, these hydrogels are more suitable as a dermal filler because the more solid nature thereof improves the mechanical properties of the filler, allowing the filler to better lift and fill a skin region. Hyaluronan polymers are typically crosslinked with a crosslinking agent to form covalent bonds between hyaluronan polymers. Such crosslinked polymers form a less water soluble hydrogel network that is more resistant to degradation, and thus requires less frequent reinjection, than the non-crosslinked hyaluronan compositions.

The present invention provides new injectable dermal filler compositions for enhancing the appearance of skin.

### SUMMARY

Accordingly, new dermal filler compositions, as well as methods of making same, are provided. Some embodiments include homogeneous hydrogel compositions prepared from hyaluronic acid and collagen. These compositions may be prepared by a method comprising crosslinking hyaluronic acid and collagen. In some embodiments, the hyaluronic acid and collagen are crosslinked under conditions in which both components are initially soluble in aqueous solution.

Some embodiments include a method for preparing a crosslinked macromolecular matrix as described herein, the method comprising: dissolving a hyaluronic acid and a collagen in an aqueous solution to form an aqueous pre-reaction solution, wherein the aqueous pre-reaction solution further comprises a salt or has a pH less than about 4; and modifying the aqueous pre-reaction solution to form a crosslinking reaction mixture comprising: the hyaluronic acid; the collagen; a water soluble carbodiimide; an N-hydroxysuccinimide or an N-hydroxysulfosuccinimide; and optionally the salt; and wherein the crosslinking reaction has a higher pH than the aqueous pre-reaction solution; and allowing the crosslinking reaction mixture to react to thereby crosslink the hyaluronic acid and the collagen.

Some embodiments include a crosslinked macromolecular matrix as injectable dermal filler comprising: a hyaluronic acid component; a collagen component derived from collagen type I or collagen type III; wherein the hyaluronic acid component is crosslinked to the collagen component by using a carbodiimide-based coupling agent, thereby forming a crosslinking component; wherein the crosslinking component comprises a plurality of crosslink units, wherein at least a portion of the crosslink units comprise an ester bond or an amide bond; and wherein the crosslinked macromolecular matrix is dispersed in an aqueous liquid in hydrogel form.

Some embodiments include a soft tissue aesthetic product comprising: an aesthetic device having a form suitable for injecting into human tissue; and a label comprising instructions to inject the aesthetic device into human tissue; wherein the aesthetic device comprises a crosslinked macromolecular matrix described herein.

Some embodiments include a method of improving an aesthetic quality of an anatomic feature of a human being comprising: injecting an aesthetic device into a tissue of the human being to thereby improve the aesthetic quality of the anatomic feature; wherein the aesthetic device comprises a crosslinked macromolecular matrix composition described herein, provided that the method is not therapeutic.

### BRIEF DESCRIPTION OF THE DRAWINGS

FIG.1A is a plot of a frequency sweep for the gel of Example 3.
FIG.1B is a plot of a strain sweep for the gel of Example 3.
FIG. 2 is an extrusion profile for the gel of Example 3.
FIG. 3 depicts scanning electron microscope images (SEM images) of the gel from Example 4 shown at 50X (A), 1,000X (B), and 40,000X (C).

### DETAILED DESCRIPTION

Crosslinked hyaluronic acid, collagen, and crosslinked collagen hydrogels, such as those used in dermal fillers, do not actively promote cellular infiltration and tissue in-growth. Similarly, collagen simply blended into hyaluronic acid hydrogels does not promote tissue integration or *de novo* tissue generation. However, some compositions described herein do promote cellular migration into the hydrogels and tissue formation within the gels when implanted *in vivo.*

Hyaluronic acid-collagen hydrogels may be synthesized by coupling a hyaluronic acid with a collagen using a coupling agent, i.e. a carbodiimide. In these hydrogels, hyaluronic acid may serve as a biocompatible water-binding component, providing bulk and isovolumetric degradation. Additionally, collagen may impart cell adhesion and signaling domains to promote cell attachment, migration, and other cell functions such as extra-cellular matrix deposition. The biopolymers form homogeneous hydrogels with tunable composition, swelling, and mechanical properties. Compositions can be made to be injectable for minimally invasive implantation through syringe and needle.

Hyaluronic acid is a non-sulfated glycosaminoglycan that enhances water retention and resists hydrostatic stresses. It is non-immunogenic and can be chemically modified in numerous fashions. Hyaluronic acid may be anionic at pH ranges around or above the pKa of its carboxylic acid groups.

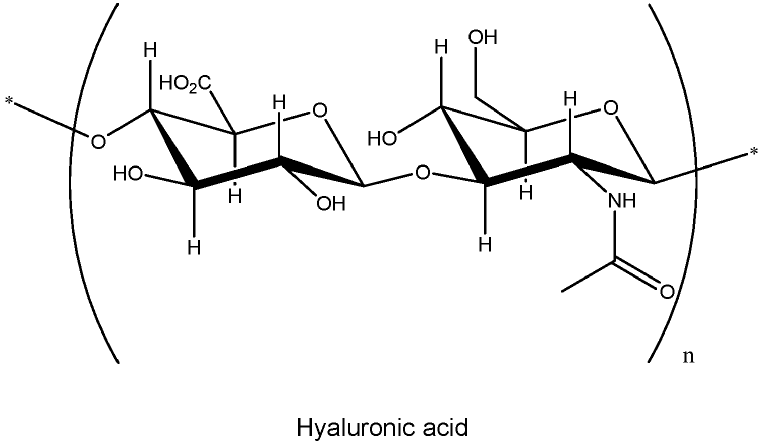

Collagen is a protein that forms fibrils and sheets that bear tensile loads. Collagen also has specific integrin-binding sites for cell adhesion and is known to promote cell attachment, migration, and proliferation. Collagen may be positively charged because of its high content of basic amino acid residues such as arginine, lysine, and hydroxylysine.

Because hyaluronic acid may be anionic and collagen may be cationic, the two macromolecules may form polyionic complexes in aqueous solution. A polyionic complex may be significantly less soluble in water than either hyaluronic acid or collagen, and thus may precipitate out of aqueous solution when the two macromolecules are together in a mixture. Furthermore, collagens are often soluble only at low pH and may precipitate from solution when brought to a pH amenable to carbodiimide coupling.

Under certain conditions, a hyaluronic acid and a collagen may be combined in an aqueous liquid in which both components are soluble. A hyaluronic acid and a collagen may then be crosslinked while both are dissolved in an aqueous solution to form a hydrogel. Reaction conditions such as the concentration of hyaluronic acid, the concentration of collagen, the pH of the solution, and salt concentration may be adjusted to help to prevent polyionic complex formation between anionic hyaluronic acid and cationic collagen. They may also help to prevent collagen microfibril formation, which results in precipitation from solution and may prevent crosslinking.

Some embodiments include a method of crosslinking hyaluronic acid and collagen. This method generally comprises a dissolution step which results in an aqueous pre-reaction solution. In a dissolution step, hyaluronic acid and collagen are dissolved in an aqueous solution that has a low pH and/or a salt to form an aqueous pre-reaction solution.

A hyaluronic acid-collagen crosslinking method further comprises an activation step. In an activation step, an aqueous pre-reaction solution is modified at least by adding a water soluble Carbodiimide and/or by increasing the pH of the solution. If needed, a salt may also be added to keep the hyaluronic acid and collagen in solution at the higher pH. Thus, a crosslinking reaction mixture comprises hyaluronic acid and collagen dissolved or dispersed in an aqueous medium, a water soluble Carbodiimide, an N-hydroxysuccinimide or an N-hydroxysulfosuccinimide, and optionally a salt, and has a higher pH than the aqueous pre-reaction solution from which it was derived. The crosslinking reaction mixture is allowed to react to thereby crosslink the hyaluronic acid and the collagen.

In some embodiments, the pH of the aqueous pre-reaction solution may be increased and a substantial amount of fiber formation may be allowed to occur in the solution before adding the water soluble coupling agent. In some embodiments, the water soluble coupling agent may be added to the aqueous pre-reaction solution before substantially any fiber formation occurs.

A crosslinking reaction mixture can react to form a crosslinked macromolecular matrix. Since reaction occurs in an aqueous solution, a crosslinked macromolecular matrix is dispersed in an aqueous liquid in hydrogel form as it is formed by a crosslinking reaction. A crosslinked macromolecular matrix may be kept in hydrogel form because, in many instances, a crosslinked macromolecular matrix may be used in hydrogel form.

In some embodiments, an aqueous pre-reaction solution or a crosslinking reaction mixture may further comprise about 10% to about 90% of an organic solvent in which hyaluronic acid has poor solubility, such as ethanol, methanol, isopropanol, or the like.

After a crosslinking reaction has occurred, the crosslinked macromolecular matrix may be particulated or homogenized through a mesh. This may help to form an injectable slurry or hydrogel. A mesh used for particulating a crosslinked macromolecular matrix may have any suitable pore size depending upon the size of particles desired. In some embodiments, the mesh may have a pore size of about 10 microns to about 100 microns, about 50 microns to about 70 microns, or about 60 microns.

A hydrogel comprising a crosslinked molecular matrix may be treated by dialysis for sterilization or other purposes. Dialysis may be carried out by placing a semipermeable membrane between the hydrogel and another liquid so as to allow the hydrogel and the liquid to exchange molecules or salts that can pass between the membrane.
A dialysis membrane may have a molecular weight cutoff that may vary. For example, the cutoff may be about 5,000 daltons to about 100,0000 daltons, about 10,000 daltons to about 30,000 daltons, or about 20,000 daltons.

The dialysis may be carried out against a buffer solution, meaning that the liquid on the other side of the membrane from the hydrogel may be a buffer solution. In some embodiments, the buffer solution may be a sterile phosphate buffer solution that may comprise phosphate buffer, potassium chloride, and/or sodium chloride. A sterile phosphate buffer solution may be substantially isosmotic with respect to human physiological fluid. Thus, when dialysis is complete, the liquid component of a hydrogel may be substantially isosmotic with respect to human physiological fluid.

In some embodiments, a crosslinked macromolecular complex further comprises an aqueous liquid. For example, the crosslinked macromolecular complex absorbs the aqueous liquid so that a hydrogel is formed. An aqueous liquid may comprise water with a salt dissolved in it, such as a phosphate buffer, sodium chloride, potassium chloride, etc. In some embodiments, an aqueous liquid may comprise water, sodium chloride at a concentration of about 100 mM to about 200 mM, potassium chloride at a concentration of about 2 mM to about 3 mM, and phosphate buffer at a concentration of about 5 mM to about 15 mM, wherein the pH of the liquid is about 7 to about 8.

A hydrogel may be used in a soft tissue aesthetic product. An aesthetic product includes any product that improves any aesthetic property of any part of an animal or human being. A soft tissue aesthetic product may comprise: an aesthetic device having a form suitable for injecting into human tissue; and a label comprising instructions to inject the aesthetic component into human tissue; wherein the aesthetic device comprises a crosslinked macromolecular matrix described herein. Some products may comprise the crosslinked macromolecular matrix in hydrogel form.

Some embodiments include a method of improving an aesthetic quality of an anatomic feature of a human being. Improving an aesthetic quality of an anatomic feature of a human being includes improving any kind of aesthetic quality including appearance, tactile sensation, etc., and improving any anatomical feature, including those of the face, limbs, breasts, buttocks, etc. Such a method may comprise: injecting an aesthetic device into a tissue of the human being to thereby improve the aesthetic quality of the anatomic feature; wherein the aesthetic device comprises a crosslinked macromolecular matrix composition described herein. In some embodiments, the crosslinked macromolecular matrix used in the product may be in hydrogel form.

In some embodiments, a hydrogel of a crosslinked macromolecular complex may have a storage modulus of about 1 Pa to about 10,000 Pa, about 50 Pa to 10,000 Pa, about 500 Pa to about 1000 Pa, about 500 Pa to about 5000 Pa, about 850 Pa, about 852 Pa, about 560 Pa, about 556 Pa, about 1000 Pa, or any value in a range bounded by, or between, any of these values.

In some embodiments, a hydrogel of a crosslinked macromolecular complex may have a loss modulus of about 1 Pa to about 500 Pa, about 10 Pa to 200 Pa, about 100 Pa to about 200 Pa, about 20 Pa, about 131 Pa, about 152 Pa, or any value in a range bounded by, or between, any of these values.

In some embodiments, a hydrogel of a crosslinked macromolecular complex may have an average extrusion force of about 10 N to about 50 N, about 20 N to 30 N, or about 25 N, when the hydrogel is forced through a 30G needle syringe by moving the plunger of a 1 mL syringe containing the hydrogel at a rate of 100 mm/min for about 11 mm, and measuring the average force from about 4 mm to about 10 mm.

A crosslinked macromolecular matrix may have tunable swelling properties based on reaction conditions and hydrogel dilution. In some embodiments, a crosslinked macromolecular matrix may have a swelling ratio of about 20 to about 200. A swelling ratio is the ratio of the weight of the crosslinked macromolecular matrix after synthesis to the weight of the crosslinked macromolecular matrix without any water. The crosslinked macromolecular matrix may have a swelling power of about 1 to about 7. The swelling power is the ratio of the weight of the crosslinked macromolecular matrix when it is saturated with water to the weight of the crosslinked macromolecular matrix after synthesis.

In a crosslinking reaction, the molecular weight of a hyaluronic acid may vary. In some embodiments, a hyaluronic acid may have a molecular weight of about 200,000 daltons to about 10,000,000 daltons, about 500,000 daltons to about 10,000,000 daltons, about 1,000,000 daltons to about 5,000,000 daltons, or about 1,000,000 daltons to about 3,000,000 daltons. When the crosslinking reaction occurs, the resulting crosslinked macromolecular product may have a hyaluronic acid component derived from the hyaluronic acid in the crosslinking reaction. Thus, the ranges recited above may also apply to the molecular weight of a hyaluronic acid component, e.g. about 200,000 daltons to about 10,000,000 daltons, about 500,000 daltons to about 10,000,000 daltons, about 1,000,000 daltons to about 5,000,000 daltons, or about 1,000,000 daltons to about 3,000,000 daltons. The term "molecular weight" is applied in this situation to a portion of the matrix even though the hyaluronic acid component may not actually be a separate molecule due to the crosslinking. In some embodiments, a higher molecular weight hyaluronic acid may result in a crosslinked molecular matrix that may have a higher bulk modulus and/or less swelling.

The concentration of hyaluronic acid in an aqueous pre-reaction solution or a crosslinking reaction mixture may vary. In some embodiments, hyaluronic acid is present at about 3 mg/mL to about 100 mg/mL, about 6 mg/mL to about 24 mg/mL, about 1 mg/mL to about 30 mg/mL, about 6 mg/mL, about 12 mg/mL, about 16 mg/mL, or about 24 mg/mL In some embodiments, higher hyaluronic acid concentration may lead to higher stiffness and/or more swelling in the crosslinked macromolecular matrix.

Collagen component derived from collagen type I or collagen type III is used in the methods and compositions described herein. Collagen type I, or collagen type III, or a combination thereof, is used. A collagen may be derived from cell culture, animal tissue, or recombinant means, and may be derived from human, porcine, or bovine sources. Some embodiments comprise collagen derived from human fibroblast culture. Some embodiments comprise collagen that has been denatured to gelatin.

Collagen concentration in an aqueous pre-reaction solution or a crosslinking reaction mixture may vary. In some embodiments, collagen may be present at a concentration of about 1 mg/mL to about 40 mg/mL, about 1 mg/mL to about 15 mg/mL, about 3 mg/mL to about 12 mg/mL, about 1.7 mg/mL, about 3 mg/mL, about 6 mg/mL, about 8 mg/mL, or about 12 mg/mL.

In some embodiments, the weight ratio of hyaluronic acid to collagen in a aqueous pre-reaction solution or a aqueous pre-reaction solution or a crosslinking reaction mixture (e.g. [wt hyaluronic acid]/[wt collagen]) may be about 0.5 to about 7, about 1 to about 5, or about 1 to about 3, or about 1 to about 2, or about 1, or about 2. When the crosslinking reaction occurs, the resulting crosslinked macromolecular product may have a collagen component derived from the collagen in the crosslinking reaction. Thus, the resulting crosslinked macromolecular matrix may have a weight ratio of hyaluronic acid component to collagen component that corresponds to the weight ratio in the crosslinking reaction, e.g. about 0.5 to about 7, about 1 to about 3, about 1 to about 2, about 1, or about 2. A higher weight ratio of hyaluronic acid to collagen may result in a crosslinked macromolecular matrix with increased swelling, decreased stiffness, and/or decreased cell adhesion.

In increase in the amount of both hyaluronic acid and collagen may result in a crosslinked macromolecular matrix with increased stiffness.

A salt may help to screen the negative charges of hyaluronic acid from positive charges of collagen, and may thus prevent precipitation of a polyionic ion complex from solution. However, high concentrations of salt may reduce the solubility of some components in solution. Thus, in some embodiments, the salt concentration of an aqueous pre-reaction solution or a crosslinking reaction mixture may be high enough to screen the charges so that the polyionic ion complex is not formed, but also low enough so that the components of the mixture remain in solution. For example, the total salt concentration of some aqueous pre-reaction solutions or crosslinking reaction mixtures may be about 10 mM to about 1 M, about 100 mM to about 300 mM, or about 150 mM. In some embodiments, a higher salt concentration may increase the efficiency of a crosslinking reaction, which may result in lower swelling and/or higher stiffness.

Some salts in an aqueous pre-reaction solution or a crosslinking reaction mixture may be non-coordinating buffers. Any non-coordinating buffer may be used that is capable of buffering the mixture and does not form coordinating complexes with coupling agents or metal atoms. Examples of suitable non-coordinating buffers may include, but are not limited to, 2-(N-morpholino)ethanesulfonic acid (MES), 3-(N-morpholino)propanesulfonic acid (MOPS), 4-(2-hydroxyethyl)-1-piperazinyl)ethanesulfonic acid (HEPES), 3-[4-(2-hydroxyethyl)-1-piperazinyl]propanesulfonic acid (HEPPS), N-cyclohexyl-2-aminoethanesulfonic acid (CHES), N-cyclohexyl-3-aminopropanesulfonic acid (CAPS), etc.

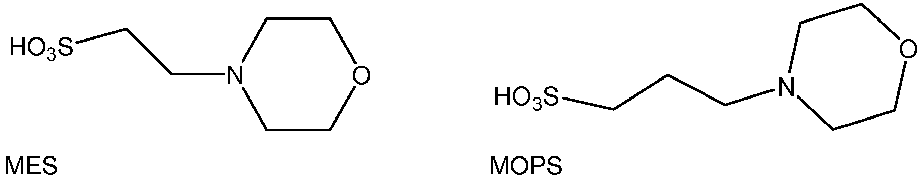

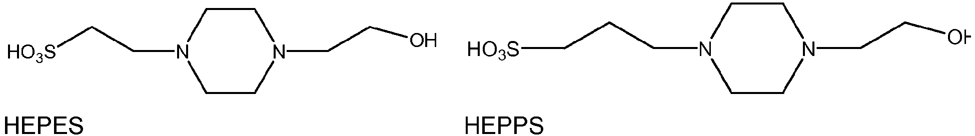

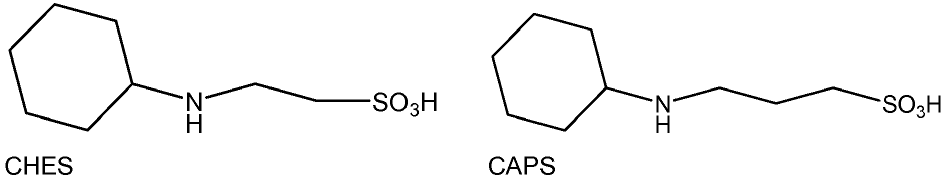

The concentration of a non-coordinating buffer may vary. For example, some aqueous pre-reaction solutions or crosslinking reaction mixtures may have a buffer concentration in a range of about 10 mM to about 1 M, about 10 mM to about 500 mM, about 20 mM to about 100 mM, or about 25 mM to about 250 mM. Some aqueous pre-reaction solutions or crosslinking reaction mixtures comprise MES at a concentration of about 20 mM to about 200 mM, about 20 mM to about 100 mM, about 100 mM, or about 180 mM.

Non-buffering salts may also be included in an aqueous pre-reaction solution or a crosslinking reaction mixture as an alternative to, or in addition, to buffering salts. Some examples may include inorganic salts such as sodium chloride, potassium chloride, lithium chloride, potassium bromide, sodium bromide, lithium bromide, and the like. The concentration of a non-buffering salt may vary. For example, some mixtures may have a non-buffering salt concentration in a range of about 10 mM to about 1 M, about 30 mM to about 500 mM, or about 50 mM to about 300 mM. In some embodiments, sodium chloride may be present at a concentration in a range of about 0.5 % w/v to about 2 % about 0.9 % w/v, about 1.6 % w/v, about 20 mM to about 1 M, about 40 mM to about 500 mM, about 50 to 300 mM, about 80 mM to about 330 mM, about 150 mM, or about 270 mM.

The pH of an aqueous pre-reaction solution may be lower than the pH of a crosslinking reaction mixture. If the salt content of the aqueous pre-reaction solution is low, the pH may be lower to enhance solubility of the hyaluronic acid and the collagen. If the salt content is higher, the pH may be higher in the aqueous pre-reaction solution. In some embodiments, the pH of the aqueous pre-reaction mixture is about 1 to about 8, about 3 to about 8, about 4 to about 6, about 4.7 to about 7.4, or about 5.4. For low salt concentrations, the pH may be about 1 to about 4 or about 1 to about 3. In some embodiments, a pH of around 5.4 may result in a crosslinked macromolecular matrix having higher stiffness and/or lower swelling.

In some embodiments, pH may be adjusted to neutral to allow collagen gelation or fiber formation before adding a coupling agent.

In some embodiments, the pH may be adjusted to neutral immediately prior to, around the time of, or after adding a coupling agent, such that collagen gelation is reduced or does not substantially occur.

The water-soluble coupling agent used can crosslink hyaluronic acid to collagen. The coupling agent is a carbodiimide-based coupling agent such as N,N'-dicyclohexylcarbodiimide (DCC), N,N'-diisopropylcarbodiimide (DIC), or 1-ethyl-3-(3-dimethylaminopropyl)carbodiimide (EDC), etc. Carbodiimide coupling agents may facilitate ester or amide bond formation without becoming part of the linkage. In other words, an ester bond or an amide bond may comprise atoms from a carboxylate group from one of hyaluronic acid or collagen, and a hydroxyl group or an amine group from the other. The concentration of a coupling agent may vary. In some embodiments, a coupling agent may be present at about 2 mM to about 150 mM, about 2 mM to about 50 mM, about 20 mM to about 100 mM, or about 50 mM. In some embodiments, the coupling agent is EDC that is present at a concentration of about 20 mM to about 100 mM, about 2 mM to about 50 mM, or about 50 mM. Increasing the carbodiimide concentration up to about 50 mM may result in a crosslinked macromolecular matrix with greater hydrogel stiffness and/or less swelling.

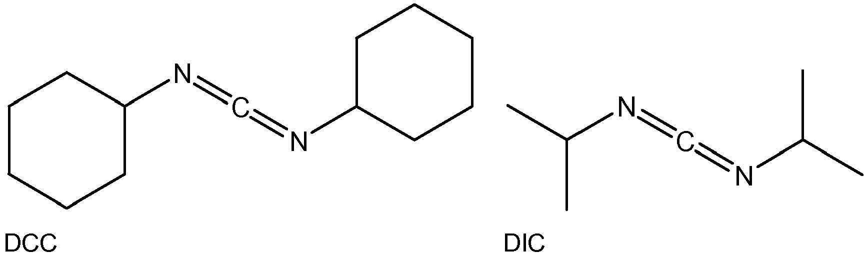

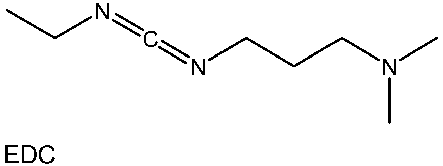

A crosslinking reaction includes any reaction hyaluronic acid is covalently linked to collagen in a plurality of (e.g. more than 1) positions. In some embodiments, a crosslinking reaction may be represented by Scheme 1 below.

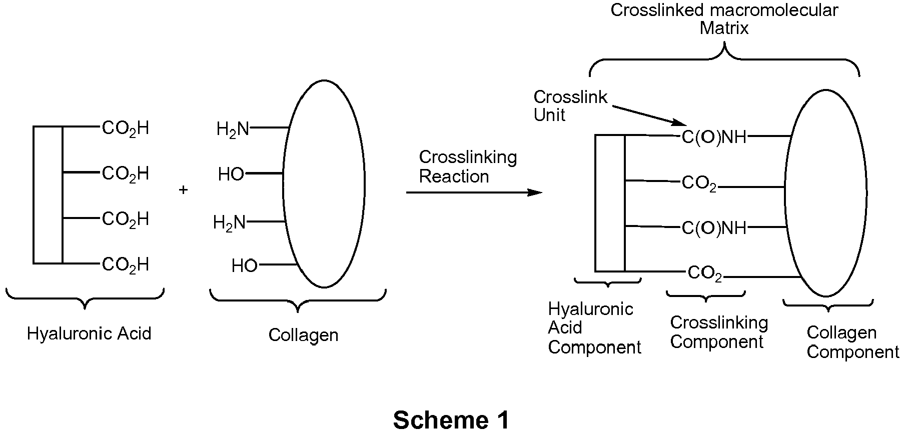

In Scheme 1, only some of the reacting functional groups are depicted, and many functional groups which may react in a crosslinking reaction, but may also remain unreacted, are not shown. For example, OH, CO₂H, -NHCOCH₃, and other groups on hyaluronic acid that are not shown may react, but may also remain unreacted. Similarly, collagen may have additional groups that may react, but may also remain unreacted, such as OH, SH, CO₂H, NH₂, etc. Additionally, fewer groups may react than those depicted.

In Scheme 1, functional groups such as CO₂H on hyaluronic acid may react with functional groups on collagen such as NH₂ and OH to form several crosslink units. The crosslink units together make up the crosslinking component. In Scheme 1, a coupling component does not become part of a crosslink unit. However, for some coupling agents, at least part of a coupling agent may incorporated into a crosslink unit. The hyaluronic acid component includes hyaluronic acid that has reacted to become part of a crosslinked macromolecular matrix. The collagen component includes collagen that has reacted to become part of a crosslinked macromolecular matrix. In addition to the crosslinking between hyaluronic acid and collagen, hyaluronic acid or collagen may be partially self-crosslinked. Thus, Scheme 1 is presented for convenience in understanding the crosslinking reaction, but does not necessarily reflect an actual chemical structure. For example, a crosslinked molecular matrix may be a network of hyaluronic acid macromolecules and collagen macromolecules, with many macromolecules crosslinked to more than one macromolecule.

As a result of a crosslinking reaction, a crosslinked macromolecular matrix may comprise a crosslinking component that crosslinks or covalently connects the hyaluronic acid component to the collagen component. As explained above, a crosslink component comprises a plurality of crosslink units, or individual covalent bonding links, between the hyaluronic acid component and the collagen component. A crosslink unit may simply be a direct bond between a hyaluronic acid component and a collagen components, so that the coupling agent may not be incorporated into the crosslinked macromolecular matrix. Alternatively, a crosslink unit may contain additional atoms or groups from the coupling agent such that at least a portion of the coupling agent may become part of the crosslinked macromolecular matrix. At least a portion of the crosslink units may comprise an ester bond or an amide bond. In some embodiments, at least a portion of the crosslink units may be -CON- or -CO₂-, where the N is a nitrogen from an amino acid residue.

An activating agent may be used to increase the rate of the crosslinking reaction and the number of crosslink units in the final product. In some embodiments, an activating agent may be a triazole such as hydroxybenzotriazole (HOBT) or 1-hydroxy-7-azabenzotriazole (HOAT); a fluorinated phenol such as pentafluorophenol; a succinimide such as N-hydroxysuccinimide (NHS) or N-hydroxysulfosuccinimide (sulfoNHS), and the like.

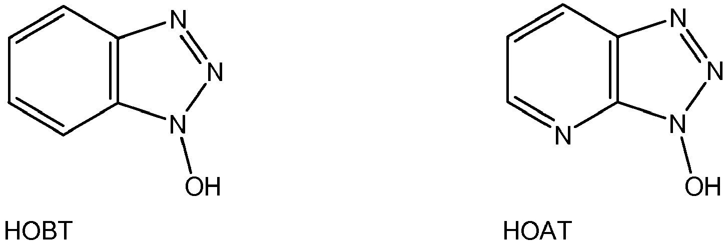

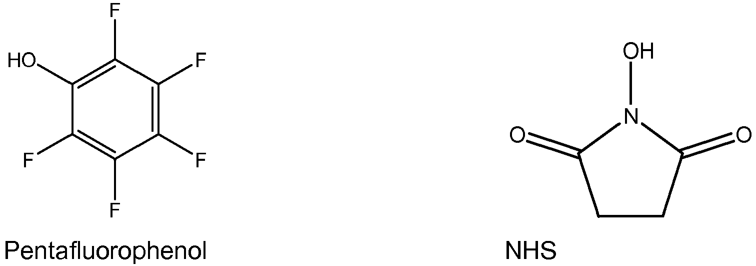

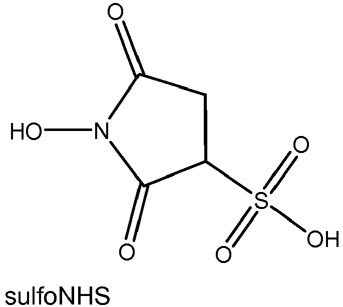

The concentration of an activating agent may vary. In some embodiments, the activating agent may have a concentration of about 2 mM to about 200 mM, about 2 mM to about 50 mM, about 20 mM to about 100 mM, or about 50 mM. In some embodments, the activating agent may be NHS or sulfoNHS is at a concentration of about 2 mM to about 50 mM. In some embodiments, the activating agent may be N-hydroxysulfosuccinimide, sodium salt, at a concentration of about 20 mM to about 100 mM, or about 50 Mm.

In some embodiments, a crosslinking reaction mixture may comprise a carbodiimide coupling agent and an activating agent. In some embodiments, the coupling agent is EDC and the activating agent is NHS or sulfoNHS. In some embodiments EDC is present at a concentration of about 2 mM to about 50 mM and NHS or sulfoNHS is present at about 2 mM to about 50 mM.

In some embodiments, a crosslinking reaction mixture may comprise hyaluronic acid at a concentration of about 3 mg/mL, human collagen type III at a concentration of about 3 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 6 mg/mL, human collagen type III at a concentration of about 6 mg/mL, 2-(N-morpholino)ethanesulfonic acid at a concentration of about 180 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 16 mg/mL of, rat collagen type I at a concentration of about 8 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 12 mg/mL, rat collagen type I at a concentration of about 12 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 12 mg/mL, rat tail collagen type I at a concentration of about 12 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.3.

In some embodiments, a crosslinking reaction mixture may comprise hyaluronic acid at a concentration of about 3 mg/mL, human collagen type I at a concentration of about 3 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 12 mg/mL, human collagen type I at a concentration of about 6 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 16 mg/mL, human collagen type I at a concentration of about 8 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 12 mg/mL, human collagen type I at a concentration of about 12 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 24 mg/mL, human collagen type I at a concentration of about 12 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 16 mg/mL, collagen at a concentration of about 8 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 1 mg/mL to about 20 mg/mL , porcine collagen type I at a concentration of about 1 mg/mL to about 15 mg/mL, 2-(N-morpholino)ethanesulfonic acid at a concentration of about 20 mM to about 200 mM, sodium chloride at a concentration of about 0.5 wt% to about 2 wt% or about 80 mM to about 330 mM, 1-ethyl-3-(3-dimethylaminopropyl)carbodiimide at a concentration of about 20 mM to about 100 mM, and N-hydroxysulfosuccinimide sodium salt at a concentration of about 20 mM to about 100 mM, wherein the solution has a pH of about 4 to about 6.

### Example 1

A solution of hyaluronic acid and collagen was created by dissolving 30 mg of 2 MDa hyaluronic acid sodium salt (Corneal) into 10 mL of 3 mg/mL collagen type III solution in 10 mM HCI (Fibrogen). 2-(N-morpholino) ethanesulfonic acid buffer salt (195.2 mg) was added to the solution along with 90 mg NaCl to form a pre-reaction solution at pH 2.5. The pH was then adjusted to 5.4 by addition of 200 µL 1 N NaOH. Next, 95.9 mg of 1-ethyl-3-(3-dimethylaminopropyl) carbodiimide HCI and 108.6 mg N-hydroxysulfosuccinimide sodium salt were added to the hyaluronic acid/collagen(III) solution and mixed thoroughly. The crosslinking reaction proceeded for 18 hrs before the gel was particulated through a 60 micron pore-sized mesh. Following sizing, the gel was sterilized by dialysis through a 20 kDa molecular-weight cut-off cellulose ester membrane against 70 % isopropanol / 30 % water for 3 hrs at 4°C. Dialysis was then continued against sterile phosphate buffer for 72 hrs at 4°C with four changes of buffer. The gel was then dispensed into syringes under aseptic conditions.

### Example 2

A solution of hyaluronic acid was created by dissolving 60 mg of 2 MDa hyaluronic acid sodium salt (Corneal) in 20 mL of 100 mM 2-(N-morpholino) ethanesulfonic acid buffer with 0.9 wt% NaCl at pH 4.7. Upon full hydration and dissolution of the hyaluronic acid, this solution was mixed with 20 mL of 3 mg/mL human collagen(III) solution in 10 mM HCI (Fibrogen). The pH of the resulting hyaluronic acid/collagen(III)solution was adjusted to 5.4 with 1 N NaOH. The solution was then lyophilized to a dry sponge and reconstituted in 10 mL of distilled water to obtain a solution of 6 mg/mL hyaluronic acid and 6 mg/mL collagen(III). Next, 192 mg of 1-ethyl-3-(3-dimethylaminopropyl) carbodiimide HCI and 217 mg of N-hydroxysulfosuccinimide sodium salt were added to the hyaluronic acid/collagen(III) solution and mixed thoroughly. The crosslinking reaction proceeded for 18 hrs before the gel was particulated through a 60 micron pore-sized mesh. Following sizing, the gel was sterilized by dialysis through a 20 kDa molecular-weight cut-off cellulose ester membrane against 70 % isopropanol / 30 % water for 3 hrs at 4°C. Dialysis was then continued against sterile phosphate buffer for 72 hrs at 4°C with four changes of buffer. The gel was then dispensed into syringes under aseptic conditions.

### Example 3

Rat tail collagen(I) (Roche) was dissolved at 20 mg/mL in 0.01 N hydrochloric acid. Hyaluronic acid, 2 MDa molecular weight, (Corneal) was dissolved at 40 mg/mL in 100 mM 2-(N-morpholino) ethanesulfonic acid buffer salt (MES) buffer with 0.9 wt% NaCl at pH 4.7. MES buffer (500 mM) at pH 6.3 was made by dissolving 43 mg of NaCl and 95 mg MES buffer salt into 100 mM MES buffer with 0.9 wt% NaCl at pH 4.7. A pre-reaction solution was created by mixing 4.2 g of the rat collagen(I) solution, 4.2 g of the hyaluronic acid solution, and 1.05 mL of the MES buffer. An activating solution was made of 114 mg of N-hydroxysulfosuccinimide sodium salt in 530 µL of 100 mM MES buffer with 0.9 wt% NaCl at pH 5.2. A coupling solution was made of 100.6 mg of 1-ethyl-3-(3-dimethylaminopropyl) carbodiimide HCI in 530 µL of 100 mM MES buffer with 0.9 wt% NaCl at pH 5.2. The reaction mixture was then created by adding 500 µL of activating solution followed by 500 µL of coupling solution to 9 g of hyaluronic acid/collagen solution. The reaction mixture was transferred to a glass vial and centrifuged for 5 min at 4000 RPM to remove air bubbles. The reaction proceeded for 18 hrs at 4°C. The gel was then particulated through a 100 micron pore-sized mesh. Following sizing, the gel was sterilized by dialysis through a 20 kDa molecular-weight cut-off cellulose ester membrane against 70 % isopropanol / 30 % water for 3 hrs at 4°C. Dialysis was then continued against sterile phosphate buffer for 72 hrs at 4°C with four changes of buffer. The gel was then dispensed into syringes under aseptic conditions.

### Example 4

Rat tail collagen(I) in 0.01 N hydrochloric acid was concentrated from 5 mg/mL to 12 mg/mL using a centrifugal filtration device with 20 kDa molecular weight cutoff. Hyaluronic acid (120 mg, 2 MDa) was added to 10 mL of the collagen solution and allowed to hydrate for 60 minutes. The solution was then homogenized by passing from syringe to syringe through a leur-leur connector. Next, 90 mg of NaCl (0.9 wt%) and 200 mg of 2-(N-morpholino) ethanesulfonic acid buffer salt (100 mM) were added to the solution and mixed. Then 98 mg of 1-ethyl-3-(3-dimethylaminopropyl) carbodiimide HCI and 111 mg of N-hydroxysulfosuccinimide sodium salt (50 mM each) were added to the solution and quickly mixed. Finally, 200 µL of 1 N NaOH was added to the solution which was mixed by syringe-to-syringe passing. The reaction solution was transferred to a glass vial and centrifuged for 5 min at 4000 RPM to remove air bubbles. The reaction proceeded for 18 hrs at 4°C. The gel was then particulated through a 100 micron pore-sized mesh. Following sizing, the gel was sterilized by dialysis through a 20 kDa molecular-weight cut-off cellulose ester membrane against 70 % isopropanol / 30 % water for 3 hrs at 4°C. Dialysis was then continued against sterile phosphate buffer for 72 hrs at 4°C with four changes of buffer. The gel was then dispensed into syringes under aseptic conditions.

### Example 5

Oscillatory parallel plate rheology was used to characterize the mechanical properties of gels using an Anton Paar MCR 301. A plate diameter of 25 mm was used at a gap height of 1 mm. A frequency sweep from 0.1 to 10 Hz at a fixed strain of 2 % with logarithmic increase in frequency was applied followed by a strain sweep between 0.1 % and 300 % at a fixed frequency of 5 Hz with logarithmic increase in strain. The storage modulus (G') and loss modulus (G") were determined from frequency sweep measurements at 5 Hz.

The gel from Example 1 had a storage modulus (G') of 505 Pa and loss modulus (G") of 70 Pa.

The gel from Example 3 had a storage modulus (G') of 2,580 Pa and loss modulus (G") of 155 Pa.

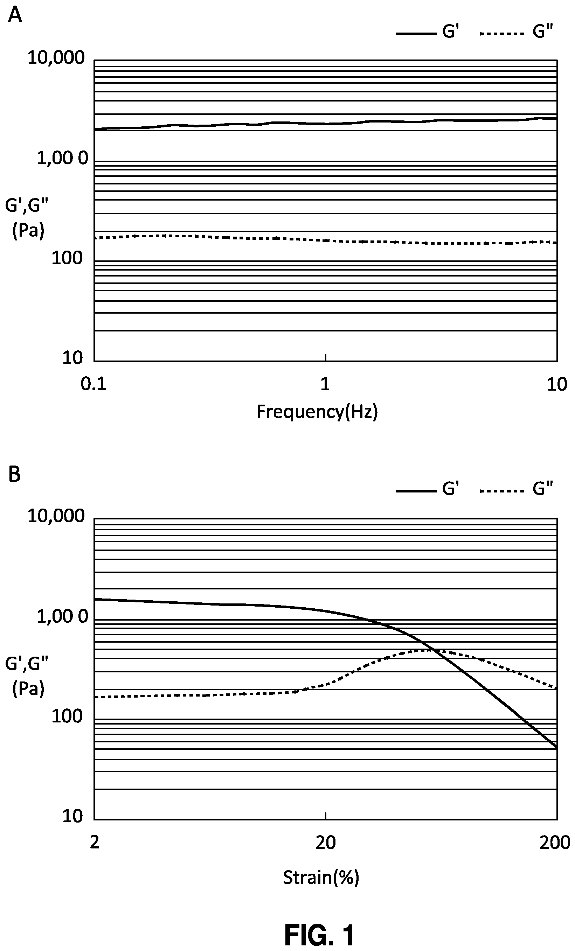

The frequency and strain sweeps for the gel from Example 3 are shown in Figure 1.

### Example 6

In order to determine the force required to extrude the gels, they were ejected from 1 mL BD syringes through 30G needles using an Instron 5564 with Bluehill 2 software. The plunger was pushed at a rate of 100 mm/min for 11.35 mm and the extrusion profile was recorded.

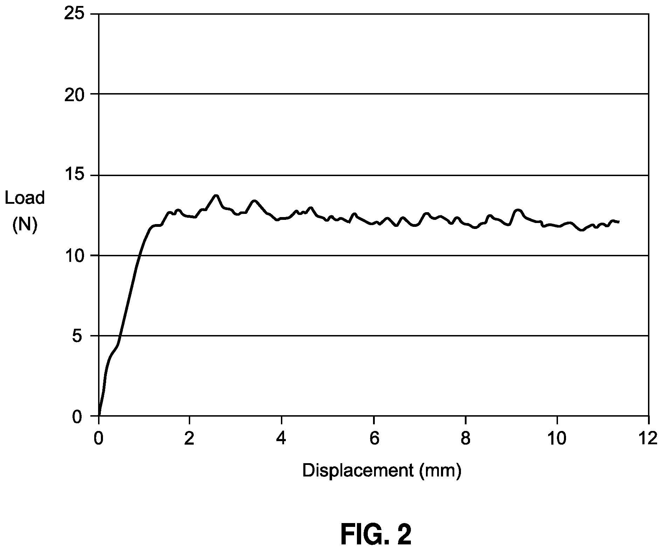

The extrusion profile through a 30G needle for gel from Example 3 is shown in Figure 2. The gel had an average extrusion force of 12.2 N from 4 through 10 mm.

### Example 7

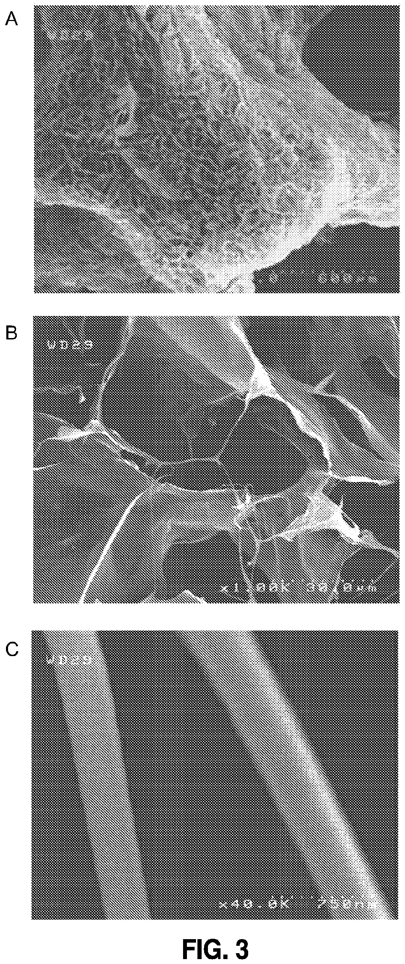

Gels were flash frozen in liquid nitrogen and dried by lyophilization. The dried sample was then imaged using a Hitachi S - 4500 scanning electron microscope (SEM). SEM images of the gel from Example 4 are shown in Figure 3 at 50X (A), 1,000X (B), and 40,000X (C). The fibrillar nature of collagen(I) is partially preserved in the hydrogel.

### Example 8

Rat tail collagen(I) in 0.01 N hydrochloric acid was concentrated from 5 mg/mL to 12 mg/mL using a centrifugal filtration device with 20 kDa molecular weight cutoff. Hyaluronic acid sodium salt (120 mg, 2 MDa) was added to 10 mL of the collagen solution and allowed to hydrate for 60 minutes. The solution was then homogenized by passing from syringe to syringe through a leur-leur connector, and 93 mg of NaCl, 201 mg of 2-(N-morpholino) ethanesulfonic acid buffer salt, and 200 µL of 1 N NaOH were added to the solution and mixed. 1-Ethyl-3-(3-dimethylaminopropyl) carbodiimide HCI (98 mg) and 111 mg of N-hydroxysulfosuccinimide sodium salt were then added and the final solution was mixed by syringe-to-syringe passing. The reaction solution was transferred to a glass vial and centrifuged for 5 min at 4000 RPM to remove air bubbles. The reaction proceeded for 16 hrs at 4°C. The gel was then particulated through a 60 micron pore-sized mesh. Following sizing, the gel was sterilized by dialysis through a 20 kDa molecular-weight cut-off cellulose ester membrane against 70 % isopropanol / 30 % water for 3 hrs at 4 °C. Dialysis was then continued against sterile phosphate buffer for 72 hrs at 4 °C with four changes of buffer. The gel was then dispensed into syringes under aseptic conditions.

### Example 9

The biocompatibility of gels was tested with a 50 µL intradermal sample injection in Sprague-Dawley rats. The implants were removed at 1 week and the explants were analyzed by histology with H&E and macrophage marker CD68 staining. Three 20X images of the CD68 staining were scored from 0-4 based on the degree of staining. These values were then averaged to give a sample score. Four samples for each gel were analyzed. The results of biocompatibility testing of Examples 3, 4, and 8 along with several commercially available dermal fillers are presented in Table 1.

**rable 1: CD68 staining scores for Examples 3, 4, and 8 as well as commercial dermal fillers.**

| | **average** | **stdev** |
|---|---|---|
| **Example 3** | 2.08 | 0.74 |
| **Example 4** | 2.58 | 0.63 |
| **Example 8** | 2.67 | 0.58 |
| **Juvederm® Ultra Plus** | 1.83 | 0.19 |
| **Juvederm® Voluma** | 1.92 | 0.42 |

### Example 10

Cytotoxicity of the gel from Example 4 was determined by NAMSA according to the Agarose Overlay Method of ISO 10993-5: Biological Evaluation of Medical Devices - Part 5: Tests for In Vitro Cytotoxicity. Triplicate wells were dosed with 0.1 mL of the gel placed on filter discs as well as 0.1 mL of 0.9 % NaCl solution placed on a filter discs and 1 cm length high density polyethylene as negative controls and 1 cm x 1 cm portion of latex as a positive control. Each was placed on an agarose surface directly overlaying a subconfluent monolayer of L929 mouse fibroblast cells. After incubating at 37 °C in 5 % CO₂ for 24 hours, the cultures were examined macroscopically and microscopically for any abnormal cell morphology and cell lysis. The test articles were scored from 0-4 based on the zone of cell lysis in proximity to the sample.

The gel from Example 4 showed no evidence of causing any cell lysis or toxicity and scored a 0 grade for cytotoxicity.

### Example 11

The intracutaneous reactivity of gel in rabbits from Example 4 was evaluated by NAMSA according to ISO 10993-10L Biological Evaluation of Medical Devices - Part 10: Tests for Irritation and Delayed-Type Hypersensitivity. Extract of the gel was prepared in 0.9 % NaCl solution (4:20 gel:saline ratio) and 0.2 mL of extract was injected intracutaneously into five separate sites on the right side of the back of each of three animals. The extract control was similarly injected on the left side of the back of each animal. The injection sites were observed at 24, 48, and 72 hours after injection for signs of erythema and edema. Erythema and edema were each scored on a scale of 0-4 at each site for each time point on each animal. The overall mean score was determined by dividing the sum of the scores by the total number of scores.

The gel from Example 4 had an overall mean score of 0, the same as the overall control group score. This indicated no signs of erythema or edema from the gel extract.

### Example 12

Hyaluronic acid sodium salt, 2 MDa molecular weight, was dissolved in human collagen(I) solution in 0.01 N hydrochloric acid (Advanced BioMatrix). Sodium chloride was added at 0.9 wt % and MES was added at 100 mM to the solution and mixed. The hyaluronic acid was allowed to hydrate for 1 hr and the solution was homogenized by syringe-to-syringe mixing. The pH of the solution was adjusted to 5.4 by addition of 1 N sodium hydroxide. 1-Ethyl-3-(3-dimethylaminopropyl) carbodiimide HCI (50 mM) and N-hydroxysulfosuccinimide sodium salt (50 mM) were added to the hyaluronic acid / collagen solution and quickly mixed by syringe-to-syringe transfer. The solution was transferred to a glass vial and centrifuged for 5 min at 4000 RPM to remove air bubbles. The resulting gel was allowed to react for 16 hrs at 4 °C. The gel was then particulated through a 100 micron pore-sized mesh. Following sizing, the gel was sterilized by dialysis through a 20 kDa molecular-weight cut-off cellulose ester membrane against 70 % isopropanol / 30 % water for 3 hrs at 4°C. Dialysis was then continued against sterile phosphate buffer, pH 7.4, for 72 hrs at 4°C with four changes of buffer. The gel was then dispensed into syringes under aseptic conditions.

This procedure was used to produce hydrogels with varying concentrations of hyaluronic acid and collagen. When required, human collagen(I) in 0.01 N hydrochloric acid was concentrated from 3 mg/mL to the desired reaction concentration in 20 kDa molecular-weight cut-off centrifugal filtration devices. A 50 mL sample of each gel was synthesized, sterilized by exposure to 70 % isopropanol, and purified by dialysis against phosphate buffer, pH 7.4. The gels synthesized are described in Table 2 along with their rheological properties. Oscillatory parallel plate rheology was used to characterize the rheological properties of gels using an Anton Paar MCR 301. A plate diameter of 25 mm was used at a gap height of 1 mm. The storage modulus (G') and loss modulus (G") were determined at 2% strain and 5 Hz.

**Table 2: Hyaluronic acid-human collagen(I) hydrogel synthesis concentrations and rheological properties**

| **Sample ID** | **[HA] (mg/mL)** | **[Col(I)] (mg/mL)** | **G' (Pa)** | **G" (Pa)** |
|---|---|---|---|---|
| **A** | 3 | 3 | 199 | 24.6 |
| **B** | 12 | 6 | 1260 | 154 |
| **C** | 16 | 8 | 2450 | 288 |
| **D** | 12 | 12 | 3160 | 420 |
| **E** | 24 | 12 | 5440 | 433 |
| **F** | 12 | 3 | 1110 | 52.2 |
| **G** | 16 | 3 | 1490 | 60.6 |
| **H** | 20 | 3 | 1770 | 49.5 |

### Example 13

In order to determine the biopolymer concentration in gels, the weight of the hydrated gel was compared to that of dried gel. A 2 mL sample of gel was weighed and dried by flash-freezing in liquid nitrogen followed by lyophilization at -50 °C and 0.02 Torr. A solution of the appropriate buffer was also weighed and dried in the same fashion to account for salt content of the gel. The total solids content of the gel was calculated by dividing the dry weight by the wet volume, assuming 1 g/mL density for the wet gel, to give a value in mg/mL. The salt solids content was then subtracted from this value to determine the biopolymer concentration in the gel.

**Table 3: Final concentrations of hyaluronic acid-human collagen(I) hydrogels**

| **Sample ID** | **[HA] (mg/mL)** | **[Col(I)] (mg/mL)** | **Final concentration (mg/mL)** |
|---|---|---|---|
| **A** | 3 | 3 | 5.3 |
| **B** | 12 | 6 | 16.3 |
| **C** | 16 | 8 | 19.4 |
| **D** | 12 | 12 | 22.6 |
| **E** | 24 | 12 | 31.6 |

### Example 14

Swelling ratios for gels were determined relative to initial water content and were measured by monitoring the increase in gel mass after equilibration with phosphate buffer. For each gel, approximately 1 mL was injected into a 15 mL Falcon tube and weighed followed by addition of 10 mL of phosphate buffered saline, pH 7.4. The gels were thoroughly mixed with the buffer and vortexed for 30 seconds. The gels were then allowed to equilibrate in the buffer for 48 hrs at 4 °C. After this time, the suspensions were centrifuged at 4000 RPM in a swinging bucket rotor for 5 minutes. The supernatant buffer was then decanted and the weight of the swollen gel was measured. The swelling ratio was determined by dividing the final weight of the swollen gel by the weight of the initial gel.

**Table 4: Swelling ratios of hyaluronic acid-human collagen(I) hydrogels**

| **Sample ID** | **[HA] (mg/mL)** | **[Col(I)] (mg/mL)** | **Swelling ratio** |
|---|---|---|---|
| **A** | 3 | 3 | 1.0 |
| **B** | 12 | 6 | 1.7 |
| **C** | 16 | 8 | 1.7 |
| **D** | 12 | 12 | 1.5 |
| **E** | 24 | 12 | 1.7 |

### Example 15

Hyaluronic acid (800 mg, 2 MDa molecular weight) was dissolved in 50 mL of 8 mg/mL porcine collagen(I) solution in 0.01 N hydrochloric acid. Sodium chloride was added at 0.9 wt% and 2-[morpholino] ethanesulfonic acid was added at 100 mM to the solution and mixed. The hyaluronic acid was allowed to hydrate for 1 hr and the solution was homogenized by syringe-to-syringe mixing. The pH of the solution was adjusted to 5.4 by addition of 1 N sodium hydroxide. 1-Ethyl-3-(3-dimethylaminopropyl) carbodiimide HCI (50 Mm) and N-hydroxysulfosuccinimide sodium salt (50 mM) were added to the hyaluronic acid/collagen solution and quickly mixed by syringe-to-syringe transfer. The solution was transferred to a glass vial and centrifuged for 5 min at 4000 RPM to remove air bubbles. The resulting gel was allowed to react for 16 hrs at 4 °C. The gel was then particulated through a 100 micron pore-sized mesh. Following sizing, the gel was sterilized by dialysis through a 20 kDa molecular-weight cut-off cellulose ester membrane against 70 % isopropanol / 30 % water for 3 hrs at 4 °C. Dialysis was then continued against sterile phosphate buffer, pH 7.4, for 72 hrs at 4 °C with four changes of buffer. The gel was then dispensed into syringes under aseptic conditions.

### Example 16

Subcutaneous bolus injections (1 mL) of sample hydrogels are performed via cannulae through a small incision on the dorsum of nude mice. Samples injected consist of crosslinked hyaluronic acid at 16 mg/mL, crosslinked human collagen(I) at 16 mg/mL, and sample B crosslinked hyaluronic acid-human collagen(I) hydrogel from Example 12. At six weeks, the volumetric duration of the samples is determined along with histological evaluation of cellular in-growth and tissue infiltration. It is found that crosslinked hyaluronic acid has 90 % volume duration, crosslinked human collagen(I) has 30 % volume duration, and crosslinked hyaluronic acid-human collagen (I) has 85 % volume duration. Histological evaluation indicated that crosslinked hyaluronic acid and crosslinked collagen has little to no tissue in-growth, whereas cells and newly deposited extracellular matrix are found throughout the hyaluronic acid-human collagen(I) sample.

## Claims

1. A crosslinked macromolecular matrix as injectable dermal filler comprising:
a hyaluronic acid component;
a collagen component derived from collagen type I or collagen type III;
wherein the hyaluronic acid component is crosslinked to the collagen component by using a carbodiimide-based coupling agent, thereby forming a crosslinking component;
wherein the crosslinking component comprises a plurality of crosslink units, wherein at least a portion of the crosslink units comprise an ester bond or an amide bond;and
wherein the crosslinked macromolecular matrix is dispersed in an aqueous liquid in hydrogel form.

2. The crosslinked macromolecular matrix of claim 1, having a weight ratio of the hyaluronic acid component to the collagen component of about 0.5 to about 7, about 1 to about 3, about 1 to about 2, about 1 to about 1, or about 2 to about 1.

3. The crosslinked macromolecular matrix of claim 1, having a weight ratio of the hyaluronic acid component to the collagen component of about 1 to about 1, or about 2 to about 1.

4. The crosslinked macromolecular matrix of claim 1, wherein the aqueous liquid comprises water, sodium chloride at a concentration of about 100 mM to about 200 mM, potassium chloride at a concentration of about 2 mM to about 3 mM, and phosphate buffer at a concentration of about 5 mM to about 15 mM, wherein the pH of the liquid is about 7 to about 8.

5. A method for preparing the crosslinked macromolecular matrix of any one of claims 1-4, wherein the method comprises the following steps:
dissolving a hyaluronic acid and a collagen in an aqueous solution to form an aqueous pre-reaction solution, wherein the aqueous pre-reaction solution further comprises a salt or has a pH less than about 4; and
modifying the aqueous pre-reaction solution to form a crosslinking reaction mixture comprising:
the hyaluronic acid;
the collagen;
a water soluble carbodiimide;
an N-hydroxysuccinimide or an N-hydroxysulfosuccinimide; and
optionally the salt; and
wherein the crosslinking reaction has a higher pH than the aqueous pre-reaction solution; and
allowing the crosslinking reaction mixture to react to thereby crosslink the hyaluronic acid and the collagen.

6. The method of claim 5, wherein modifying the aqueous pre-reaction solution to form a crosslinking reaction mixture comprises (i) increasing the pH of the aqueous pre-reaction solution and allowing fiber formation to occur before adding the water soluble carbodiimide; or (ii) adding the water soluble carbodiimide to the aqueous pre-reaction solution before any fiber formation occurs.

7. The method of claim 5, wherein the salt comprises sodium chloride at a concentration of about 80 mM to about 330 mM in the crosslinking reaction mixture.

8. The method of claim 5, wherein the water soluble carbodiimide is 1-ethyl-3-(3-dimethylaminopropyl)carbodiimide at a concentration of about 20 mM to about 100 mM in the crosslinking reaction mixture.

9. The method of claim 5, wherein the crosslinking reaction mixture further comprises a non-coordinating buffer having a concentration of about 10 mM to about 1 M.

10. The method of claim 5, further comprising particulating the crosslinked macromolecular matrix through a mesh having a pore size of about 10 microns to 100 microns.

11. The method of claim 5, further comprising sterilizing the crosslinked molecular matrix by dialysis;
wherein the dialysis is against a sterile phosphate buffer solution comprising phosphate buffer, potassium chloride, and sodium chloride, wherein the sterile phosphate buffer solution is substantially isosmotic with respect to human physiological fluid; and
wherein the dialysis is through a membrane having a molecular weight cutoff of about 5,000 daltons to about 100,0000 daltons.

12. A crosslinked macromolecular matrix prepared by the process of any one of claims 5-11.

13. A soft tissue aesthetic product comprising:
an aesthetic device having a form suitable for injecting into human tissue; and
a label comprising instructions to inject the aesthetic device into human tissue;
wherein the aesthetic device comprises the crosslinked macromolecular matrix of any one of claims 1-4 or claim 12.

14. The product of claim 13, wherein the crosslinked macromolecular matrix has a weight ratio of the hyaluronic acid component to the collagen component of about 1 to about 3.

15. A method of improving an aesthetic quality of an anatomic feature of a human being comprising:
injecting an aesthetic device into a tissue of the human being to thereby improve the aesthetic quality of the anatomic feature;
wherein the aesthetic device comprises the crosslinked macromolecular matrix of any one of claims 1-4 or claim 12;
provided that the method is not therapeutic.

## Patentansprüche

1. Vernetzte makromolekulare Matrix als injizierbarer dermaler Füllstoff, umfassend:
eine Hyaluronsäurekomponente;
eine Kollagenkomponente abgeleitet von Kollagen Typ I oder Kollagen Typ III;
wobei die Hyaluronsäurekomponente mit der Kollagenkomponente unter Verwendung eines Kupplungsmittels auf Carbodiimidbasis vernetzt ist und so eine vernetzte Komponente bildet;
wobei die vernetzte Komponente mehrere Vernetzungseinheiten umfasst, wobei mindestens ein Teil der Vernetzungseinheiten eine Esterbindung oder eine Amidbindung umfasst; und
wobei die vernetzte makromolekulare Matrix in einer wässrigen Flüssigkeit Hydrogel-artig verteilt ist.

2. Vernetzte makromolekulare Matrix nach Anspruch 1, die ein Gewichtsverhältnis der Hyaluronsäurekomponente zu der Kollagenkomponente von ungefähr 0,5 zu ungefähr 7, ungefähr 1 zu ungefähr 3, ungefähr 1 zu ungefähr 2, ungefähr 1 zu ungefähr 1 oder ungefähr 2 zu ungefähr 1 aufweist.

3. Vernetzte makromolekulare Matrix nach Anspruch 1, die ein Gewichtsverhältnis der Hyaluronsäurekomponente zu der Kollagenkomponente von ungefähr 1 zu ungefähr 1 oder ungefähr 2 zu ungefähr 1 aufweist.

4. Vernetzte makromolekulare Matrix nach Anspruch 1, wobei die wässrige Flüssigkeit Wasser, Natriumchlorid in einer Konzentration von ungefähr 100 mM bis ungefähr 200 mM, Kaliumchlorid in einer Konzentration von ungefähr 2 mM bis ungefähr 3 mM und Phosphatpuffer in einer Konzentration von ungefähr 5 mM bis ungefähr 15 mM umfasst, wobei der pH der Flüssigkeit ungefähr 7 bis ungefähr 8 beträgt.

5. Verfahren zur Herstellung der vernetzten makromolekularen Matrix nach mindestens einem der Ansprüche 1-4, wobei das Verfahren die folgenden Schritte umfasst:
eine Hyaluronsäure und ein Kollagen werden in einer wässrigen Lösung so gelöst, dass eine wässrige Vorreaktionslösung gebildet wird, wobei die wässrige Vorreaktionslösung ferner ein Salz umfasst oder einen pH von weniger als ungefähr 4 aufweist; und
die wässrige Vorreaktionslösung wird so modifiziert, dass eine Vernetzungsreaktionsmischung gebildet wird, umfassend:
die Hyaluronsäure;
das Kollagen;
ein wasserlösliches Carbodiimid;
ein N-Hydroxysuccinimid oder ein N-Hydroxysulfosuccinimid; und
optional das Salz; und
wobei die Vernetzungsreaktionsmischung einen höheren pH aufweist als die wässrige Vorreaktionslösung; und
die Vernetzungsreaktionsmischung wird so umgesetzt, dass die Hyaluronsäure und das Kollagen vernetzt werden.

6. Verfahren nach Anspruch 5, bei dem das Modifizieren der wässrigen Vorreaktionslösung zur Bildung einer Vernetzungsreaktionsmischung es umfasst, dass (i) der pH der wässrigen Vorreaktionslösung erhöht wird und Faserbildung stattfinden gelassen wird bevor das wasserlösliche Carbodiimid hinzugefügt wird; oder (ii) das wasserlösliche Carbodiimid zu der wässrigen Vorreaktionslösung hinzugefügt wird bevor jegliche Faserbildung stattfindet.

7. Verfahren nach Anspruch 5, bei dem das Salz Natriumchlorid in einer Konzentration von ungefähr 80 mM bis ungefähr 330 mM in der Vernetzungsreaktionsmischung umfasst.

8. Verfahren nach Anspruch 5, bei dem das wasserlösliche Carbodiimid 1-Ethyl-3-(3-dimethylaminopropyl)carbodiimid in einer Konzentration von ungefähr 20 mM bis ungefähr 100 mM in der Vernetzungsreaktionsmischung ist.

9. Verfahren nach Anspruch 5, bei dem die Vernetzungsreaktionsmischung ferner einen nichtkoordinierenden Puffer mit einer Konzentration von ungefähr 10 mM bis ungefähr 1 M umfasst.

10. Verfahren nach Anspruch 5, ferner umfassend, dass die vernetzte makromolekulare Matrix durch ein Netz mit einer Porengröße von ungefähr 10 Mikrometer bis 100 Mikrometer partikelförmig gemacht wird.

11. Verfahren nach Anspruch 5, ferner umfassend, dass die vernetzte makromolekulare Matrix durch Dialyse sterilisiert wird;
wobei die Dialyse gegen eine sterile Phosphatpufferlösung, die Phosphatpuffer, Kaliumchlorid und Natriumchlorid umfasst, stattfindet, wobei die sterile Phosphatpufferlösung im Wesentlichen isoosmotisch in Bezug auf menschliche physiologische Flüssigkeit ist; und
wobei die Dialyse durch eine Membran mit einem Molekulargewicht-Cutoff von ungefähr 5.000 Dalton bis ungefähr 100.0000 Dalton stattfindet.

12. Vernetzte makromolekulare Matrix, hergestellt durch das Verfahren nach mindestens einem der Ansprüche 5-11.

13. Ästhetisches Weichgewebeprodukt, umfassend:
eine ästhetische Vorrichtung mit einer Form, die zur Injektion ins menschliche Gewebe geeignet ist; und
ein Label, das Anweisungen für die Injektion ins menschliche Gewebe umfasst;
wobei die ästhetische Vorrichtung die vernetzte makromolekulare Matrix nach mindestens einem der Ansprüche 1-4 oder Anspruch 12 umfasst.

14. Produkt nach Anspruch 13, wobei die vernetzte makromolekulare Matrix ein Gewichtsverhältnis der Hyaluronsäurekomponente zu der Kollagenkomponente von ungefähr 1 zu ungefähr 3 aufweist.

15. Verfahren zur Verbesserung einer ästhetischen Qualität eines anatomischen Merkmals eines Menschen, umfassend:
eine ästhetische Vorrichtung wird in ein Gewebe des Menschen injiziert und so die ästhetische Qualität des anatomischen Merkmals verbessert;
wobei die ästhetische Vorrichtung die vernetzte makromolekulare Matrix nach mindestens einem der Ansprüche 1-4 oder Anspruch 12 umfasst;
vorausgesetzt, dass das Verfahren nicht therapeutisch ist.

## Revendications

1. Matrice macromoléculaire réticulée en tant que produit de comblement dermique injectable comprenant :
un composant acide hyaluronique ;
un composant collagène dérivé du collagène de type 1 ou du collagène de type III ;
dans laquelle le composant acide hyaluronique est réticulé au composant collagène par le biais d'un agent de couplage à base de carbodiimide, formant ainsi un composant de réticulation ;
dans laquelle le composant de réticulation comprend une pluralité de motifs de réticulation, dans laquelle au moins une partie des motifs de réticulation comprend une liaison ester ou une liaison amide ; et
dans laquelle la matrice macromoléculaire réticulée est dispersée dans un liquide aqueux sous forme d'hydrogel.

2. Matrice macromoléculaire réticulée selon la revendication 1, ayant un rapport pondéral du composant acide hyaluronique sur le composant collagène d'environ 0,5 sur environ 7, d'environ 1 sur environ 3, d'environ 1 sur environ 2, d'environ 1 sur environ 1 ou d'environ 2 sur environ 1.

3. Matrice macromoléculaire réticulée selon la revendication 1, ayant un rapport pondéral du composant acide hyaluronique sur le composant collagène d'environ 1 sur environ 1 ou d'environ 2 sur environ 1.

4. Matrice macromoléculaire réticulée selon la revendication 1, dans laquelle le liquide aqueux comprend de l'eau, du chlorure de sodium à une concentration d'environ 100 mM à environ 200 mM, du chlorure de potassium à une concentration d'environ 2 mM à environ 3 mM et un tampon phosphate à une concentration d'environ 5 mM à environ 15 mM, dans laquelle le pH du liquide est d'environ 7 à environ 8.

5. Procédé de préparation de la matrice macromoléculaire réticulée selon l'une quelconque des revendications 1 à 4, dans lequel le procédé comprend les étapes suivantes :
dissolution d'un acide hyaluronique et d'un collagène dans une solution aqueuse pour former une solution pré-réactionnelle aqueuse, dans lequel la solution pré-réactionnelle aqueuse comprend en outre un sel ou a un pH inférieur à environ 4 ; et
modification de la solution pré-réactionnelle aqueuse pour former un mélange réactionnel de réticulation comprenant :
l'acide hyaluronique ;
le collagène ;
un carbodiimide hydrosoluble ;
un N-hydroxysuccinimide ou un N-hydroxysulfosuccinimide ; et
éventuellement le sel ; et
dans lequel la réaction de réticulation a un pH supérieur à celui de la solution pré-réactionnelle aqueuse ; et
la mise en réaction du mélange réactionnel de réticulation pour ainsi réticuler l'acide hyaluronique et le collagène.

6. Procédé selon la revendication 5, dans lequel la modification de la solution pré-réactionnelle aqueuse pour former un mélange réactionnel de réticulation comprend (i) l'augmentation du pH de la solution pré-réactionnelle aqueuse et la formation de fibres avant l'ajout du carbodiimide hydrosoluble ; ou (ii) l'ajout du carbodiimide hydrosoluble à la solution pré-réactionnelle aqueuse avant toute formation de fibres.

7. Procédé selon la revendication 5, dans lequel le sel comprend du chlorure de sodium à une concentration d'environ 80 mM à environ 330 mM dans le mélange réactionnel de réticulation.

8. Procédé selon la revendication 5, dans lequel le carbodiimide hydrosoluble est le 1-éthyl-3-(3-diméthylaminopropyl)carbodiimide à une concentration d'environ 20 mM à environ 100 mM dans le mélange réactionnel de réticulation.

9. Procédé selon la revendication 5, dans lequel le mélange réactionnel de réticulation comprend en outre un tampon non coordinant ayant une concentration d'environ 10 mM à environ 1 M.

10. Procédé selon la revendication 5, comprenant en outre la transformation en particules de la matrice macromoléculaire réticulée à travers une maille ayant une taille de pore d'environ 10 µm à 100 µm.

11. Procédé selon la revendication 5, comprenant en outre la stérilisation de la matrice moléculaire réticulée par dialyse ;
dans lequel la dialyse est contre une solution de tampon phosphate stérile comprenant un tampon phosphate, du chlorure de potassium et du chlorure de sodium, dans lequel la solution de tampon phosphate stérile est essentiellement isosmotique par rapport à un fluide physiologique humain ; et
dans lequel la dialyse est effectuée à travers une membrane ayant un seuil de poids moléculaire d'environ 5 000 daltons à environ 100 0000 daltons.

12. Matrice macromoléculaire réticulée préparée par le procédé selon l'une quelconque des revendications 5 à 11.

13. Produit esthétique pour tissu mou comprenant :
un dispositif esthétique ayant une forme appropriée pour une injection dans un tissu humain ; et
une étiquette comprenant les instructions d'injection du dispositif esthétique dans un tissu humain ;
dans lequel le dispositif esthétique comprend la matrice macromoléculaire réticulée selon l'une quelconque des revendications 1 à 4 ou la revendication 12.

14. Produit selon la revendication 13, dans lequel la matrice macromoléculaire réticulée a un rapport pondéral du composant acide hyaluronique au composant collagène d'environ 1 à environ 3.

15. Procédé d'amélioration d'une qualité esthétique d'une caractéristique anatomique d'un être humain comprenant :
l'injection d'un dispositif esthétique dans un tissu de l'être humain pour ainsi améliorer la qualité esthétique de la caractéristique anatomique ;
dans lequel le dispositif esthétique comprend la matrice macromoléculaire réticulée selon l'une quelconque des revendications 1 à 4 ou la revendication 12 ;
à condition que le procédé ne soit pas thérapeutique.
